(19) Europäisches Patentamt
European Patent Office
Office européen des brevets

(11) Publication number: **0 172 614 B1**

(12) **EUROPEAN PATENT SPECIFICATION**

(45) Date of publication of patent specification: 18.01.89

(51) Int. Cl.[4]: C 07 D 207/16, C 07 C 153/07, C 07 D 277/06, C 12 P 41/00

(21) Application number: 85304045.9

(22) Date of filing: 07.06.85

(54) **Process for producing optically active carboxylic acid amides.**

(30) Priority: 16.08.84 JP 169932/84

(43) Date of publication of application: 26.02.86 Bulletin 86/09

(45) Publication of the grant of the patent: 18.01.89 Bulletin 89/03

(84) Designated Contracting States: DE FR GB IT NL SE

(56) References cited:
EP-A-0 008 831
EP-A-0 008 833
DE-A-2 703 828
DE-A-2 845 499
US-A-4 283 407
HOUBEN-WEYL "Methoden der organischen Chemie", 4th edition, vol. IX, 1955: "Schwefel-, Selen-, Tellur-Verbindungen" GEORG THIEME VERLAG, Stuttgart pages 749-756
CHEMICAL ABSTRACTS, vol. 78, no. 19, May 14, 1973, Columbus, Ohio, USA VASIL'EVA, T.P. et al. "Preparation of beta-chlorosulfenylcarboxylic acids and 1,2-dithional-3-ones" pages 431, 432, abstract-no. 123 945e

(73) Proprietor: MITSUBISHI RAYON CO. LTD.
3-19, Kyobashi 2-chome Chuo-Ku
Tokyo 104 (JP)

(72) Inventor: Sakimae, Akihiro
2-6-404, Kurokawa 3-chome
Ohtake-shi Hiroshima 739-06 (JP)
Inventor: Kagawa, Yuri
2-19, Kamitenmachi Nishi-ku
Hiroshima-shi Hiroshima 733 (JP)
Inventor: Numazawa, Ryozo
2-6-304, Kurokawa 3-chome
Ohtake-shi Hiroshima 739-06 (JP)
Inventor: Onishi, Hisao
1665-219, Ohnocho
Saeki-gun Hiroshima 739-04 (JP)

(74) Representative: Woodin, Anthony John et al
Fitzpatricks Europe House Box No. 88 World Trade Centre East Smithfield
London E1 9AA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel process for producing optically active carboxylic acid amides of the formula;

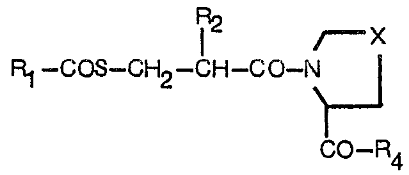

(I)

wherein $R_1$ is an alkyl group, an aralkyl group or an aryl group, $R_2$ is an alkyl group, $R_4$ is a hydroxyl group, an amino group or an alkoxy group, and X is a methylene group or a sulfur atom.

The optically active carboxylic acid amides of the formula (I) have varieties of physiological activities and are useful as medicine such as antihypertension agent.

Hitherto, as a process for producing the optically active carboxylic acid amides mentioned above, a process is known wherein physicochemical resolution of racemic carboxylic acids, such as 3-thiobenzoyl-2-alkylpropionic acid, with an optically resolving agent is effected to produce optically active carboxylic acids, and then the acids are allowed to react with amine derivatives, such as proline (EP—8831 and WPI 80—76079c/43).

However, this process is not satisfactory yet from an economical point of view, since there are drawbacks in that a large amount of an expensive resolving agent is needed in the steps of the resolution for preparing optically active carboxylic acides, that the resolution is very complicated, and that a small amount of the resolving agent is liable to be left removed as an impurity in the product. Appearance of more expedient and economical process without such drawbacks has been desired for producing optically active carboxylic acids.

After having been studying extensively processes for producing optically active carboxylic acids requiring no such resolving agents and processes for producing optically active carboxylic acid amides from the produced optically active carboxylic acid obtained above, the present inventors have succeeded in finding that optically active carboxylic acids are easily prepared by use of enzymes or microorganisms, and that the optically active carboxylic acid amides are obtainable by allowing the optically active carboxylic acid to react with amine compounds.

According to present invention, it provides a process for producing optically active carboxylic acid amides of the formula;

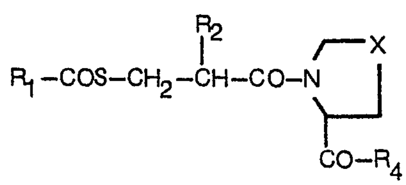

(I)

wherein $R_1$, $R_2$, $R_4$ and X are as defined above, which comprises allowing carbothioic acids of the formula;

$$R_1—COSH \qquad (II)$$

wherein $R_1$ is as defined above, to react with unsaturated carboxylic acid esters of the formula;

$$CH_2 = \overset{\displaystyle R_2}{\overset{|}{C}}—COO—R_3 \qquad (III)$$

wherein $R_2$ and $R_3$ each represents an alkyl group, to obtain esters of the formula;

$$R_1—COS—CH_2—\overset{\displaystyle R_2}{\overset{|}{C}H}—COO—R_3 \qquad (IV)$$

wherein $R_1$, $R_2$ and $R_3$ are as defined above, biochemically and asymmetrically hydrolyzing the esters of the formula (IV) above to obtain optically active carboxylic acids of the formula;

$$R_1—COS—CH_2—\overset{\displaystyle R_2}{\overset{|}{C}H}—COOH \qquad (V)$$

wherein $R_1$ and $R_2$ are as defined above, and allowing the optically active carboxylic acids of the formula (V) above to react with amine compounds of the formula;

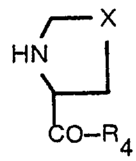

(VI)

wherein $R_4$ and X are as defined above.

As for the substituent $R_1$, there may be exemplified methyl, ethyl and propyl groups for an alkyl group, benzyl group for an aralkyl group and phenyl group for an aryl group. The alkyl group for the substituents $R_2$ and $R_3$ may be, for example, methyl group or ethyl group.

In practicing the process of the present invention, the esters of the formula (IV) above are prepared first by allowing the carbothioic acids of the formula (II) to react with the unsaturated carboxylic acids of the formula (III). It is preferable to carry out this reaction step in the presence of a polymerization inhibitor, such as hydroquinone, in order to inhibit the unsaturated carboxylic acids of the formula (III) from polymerization thereof. This reaction may be accelerated by raising the reaction temperature.

The reaction product of the formula (IV) may be isolated and purified by distillation. Examples of the esters of the formula (IV) prepared by this reaction are methyl 3-acetylthio-2-methylpropionate, methyl 3-benzoylthio-2-methylpropionate and methyl 3-phenylacetylthio-2-methylpropionate.

The esters of the formula (IV) are then asymmetrically hydrolyzed by use of a biochemical process until the optically active carboxylic acids of the formula (V) are obtained. The biochemical and asymmetrical hydrolysis of the esters is carried out by using enzymes or microorganisms having an ability to asymmetrically hydrolyze the esters. As the microorganisms having an ability to asymmetrically hydrolyze the esters, genuses Torulopsis such as Torulopsis gropengiesseri, Aspergillus such as Aspergillus sojae IAM 2703, Bacillus such as Bacillus subtilis var niger IFO 3108, Candida such as Candida rugosa IFO 0750, Candida parapsilosis IFO 0708 and Candida utilis IFO 0396, Botrytis such as Botrytis cinerea IAM 5126, Ophiobolus such as Ophiobolus miyabeanus IAM 8053, Chaetomium such as Chaetomium semispirale IFO 8363, Cladosporium such as Cladosporium resinae f. avellaneum HUT 5050, Pseudomonas such as Pseudomonas ovalis IAM 1046 and IAM 1158, Pseudomonas putida IFO 3738 and IFO 12996 and Pseudomonas fluorescens IFO 3081 and IFO 12055, Escherichia such as Escherichia coli IFO 13500, Staphylococcus such as Staphylococcus aureus IFO 12732, Alcaligenes such as Alcaligenes faecalis IFO 13111, Streptomyces such as Streptomyces griseus IFO 3355, Streptomyces clavuligerus IFO 13307, Rhodococcus such as Rhodococcus erythropolis IFO 12538, IFO 12539 and IFO 12540, Nocardia such as Nocardia asteroides IFO 3384, Mycobacterium such as Mycobacterium phlei IFO 13160 and Agrobacterium such as Agrobacterium radiobacter IFO 12607 can be used. And as enzymes, lipase, esterase, chymotrypsin and pancreatin can be used.

In conducting the hydrolysis of the esters by using microorganisms, the esters may be added to a culture medium at the beginning of or during the culturing. Instead of the above, ester may be added to the culture medium where the microorganisms have already been cultured. Alternatively, microorganism cells proliferated may be collected by, for example, centrifugation from the culture medium, and then added to the reaction medium containing the esters. In this alternative case, as a matter of convenience, cells may be used in the form of dried cells such as lyophilized or spray-dried ones or cells treated with organic solvents such as acetone or toluene. In addition, cells may be used in the form of treated cell materials such as destructed cells, or cell extract. When enzymes are used, the enzymes together with the esters are added to a reaction medium.

As the reaction medium, deionized water or a buffer solution may be used. A preferred concentration of the ester in the reaction medium or in the culture medium is 0.01—50% by weight. The ester may be added in the form of a suspension in water. An organic solvent, such as methanol or acetone, may be added to the reaction medium in order to increase the solubility of the ester. The reaction is usually conducted, for example, under pH 2—11, preferably 5—8 and at a temperature of 5—50°C, although the pH and the temperature may vary depending upon varieties of the microorganisms and enzymes. The pH of the reaction mixture changes as the reaction proceeds accompanying formation of the carboxylic acids. It is preferred to control the pH of the mixture in order to maintain at the optimum level by adding a neutralizing agent thereto.

The isolation of the optically active carboxylic acids from the reaction mixture and purification thereof can be carried out according to any of the conventional methods for example, extraction, distillation, column chromatography and the like.

Examples of the optically active carboxylic acids prepared by this reaction are D- or L-isomer of 3-acetylthio-2-methylpropionic acid, 3-benzoylthio-2-methylpropionic acid and 3-phenylacetylthio-2-methylpropionic acid.

The thus-prepared optically active carboxylic acids of the formula (V) are then allowed to react with the amine compounds of the formula (VI). Although the optically active carboxylic acids of the formula (V) may be used as they are, i.e., in the form of the free acid, it is preferable to use them in the form of reactive

derivatives thereof, for example, acid chloride, acid bromide, acid anhydride, mixed acid anhydride and the like. These reactive derivatives are prepared by allowing the optically active carboxylic acids of the formula (V) to react with, for example, inorganic acid halides, sulfonic acid halides or alkyl halocarbonates. For this purpose, thionyl chloride, phosphorus oxychloride and the like are used as the inorganic acid halides; mesyl chloride, tosyl chloride and the like as the sulfonic acid halides; and ethyl chlorocarbonate, isobutyl chlorocarbonate and the like as the alkyl halocarbonates.

As the compounds of the formula (VI), there may be exemplified proline, thioproline, their esters such as methyl, ethyl and t-butyl esters, and their amides such as prolinamide and thioprolinamide. These compounds may be in the form of a mixture of their D- and L-isomers, or they may be used in their optically active form.

The reaction of the compounds of the formula (V) with the compounds of the formula (VI) is preferably conducted in a solvent. As the solvent, for example, any of methylene chloride, chloroform, ether, dioxane, toluene, dimethylformamide and tetrahydrofuran may be used. They may be mixed with water. The reaction is preferably conducted in the presence of inorganic bases such as potassium carbonate, sodium carbonate, potassium hydroxide, sodium hydroxide, sodium hydrogencarbonate and the like, or organic bases such as trimethylamine, N,N-dimethylaniline, N,N-dimethylbenzylamine and the like. The reaction temperature is usually −50°C or higher, preferably in the range of from −20°C to +50°C.

The following compounds are examples of the optically active carboxylic acid amides prepared as above:

1-(3-Acetylthio-2-D-methylpropanoyl)-L-proline,
1-(3-Acetylthio-2-D-methylpropanoyl)-D-proline,
1-(3-Acetylthio-2-D-methylpropanoyl)-DL-proline,
1-(3-Acetylthio-2-D-methylpropanoyl)-L-thioproline,
1-(3-Acetylthio-2-D-methylpropanoyl)-D-thioproline,
1-(3-Acetylthio-2-D-methylpropanoyl)-DL-thioproline,
1-(3-Acetylthio-2-D-methylpropanoyl)-L-prolinamide,
1-(3-Acetylthio-2-D-methylpropanoyl)-L-thioprolinamide,
t-Butyl ester of 1-(3-acetylthio-2-D-methylpropanoyl)-L-proline,
1-(3-Benzoylthio-2-D-methylpropanoyl)-L-proline,
1-(3-Benzoylthio-2-D-methylpropanoyl)-DL-proline,
1-(3-Benzoylthio-2-D-methylpropanoyl)-L-thioproline,
1-(3-Benzoylthio-2-D-methylpropanoyl)-DL-thioproline,
1-(3-Benzoylthio-2-D-methylpropanoyl)-L-prolinamide,
1-(3-Benzoylthio-2-D-methylpropanoyl)-L-thioprolinamide,
Methyl ester of 1-(3-benzoylthio-2-D-methylpropanoyl)-L-proline,
Methyl ester of 1-(3-benzoylthio-2-D-methylpropanoyl)-L-thioproline,
1-(3-Phenylacetylthio-2-D-methylpropanoyl)-L-proline,
1-(3-Acetylthio-2-L-methylpropanoyl)-DL-proline,
1-(3-Benzoylthio-2-L-methylpropanoyl)-DL-proline,
L-(3-Phenylacetylthio-2-L-methylpropanoyl)-DL-proline.

According to the present invention, the intermediate optically active carboxylic acids, are easily obtained, and the optically active carboxylic acid amides of the formula (I) are commercially prepared therefrom with great advantages.

The following examples are given to illustrate the present invention more precisely, but it is not intended to limit the present invention thereto.

## Example

(A) Synthesis of methyl 3-acetylthio-2-DL-methylpropionate:

One hundred grams of methyl methacrylate containing 200 ppm of hydroquinone were added dropwise to 123 g of thioacetic acid for about 2 hours, while the thioacetic acid was warmed to 50°C and stirred. Thereafter, the temperature of the mixture was raised to 90°C and the mixture was allowed to react for about 4 hours. After the reaction was over, the reaction mixture was distilled under reduced pressure and a fraction boiling at 119°—128°C at 3—4 mm Hg was collected and cooled to give 152 g of methyl 3-acetylthio-2-DL-methylpropionate.

(B) Synthesis of 3-acetylthio-2-D-methylpropionic acid:

Cells of *Pseudomonas fluorescens* IFO 12055 were collected by centrifugation after the proliferation in a liquid medium (pH 7) containing meat extract (1.0% by weight), peptone (1.0% by weight) and sodium chloride (0.5% by weight). After 10.1 g (in terms of dried cell body weight) of the cells were suspended in 1.5 l of an M/2 phosphoric acid buffer solution (pH 7.0), 75 ml of methyl 3-acetylthio-2-DL-methylpropionate was added to the suspension and allowed to react at 30°C for 48 hours. After the reaction was over, the cells were removed from the reaction mixture by centrifugation, and the unaltered ester was removed by extraction with an equal volume of ethyl acetate.

After the pH of the resulting aqueous layer was controlled to 2 by addition of sulfuric acid, 30.2 g of 3-acetylthio-2-D-methylpropionic acid was obtained by extraction with an equal volume of ethyl acetate. The specific rotation of the product $[\alpha]_D^{25}$ was −53.8° (C = 1.1, $CHCl_3$).

(C) Synthesis of 1-(3-acetylthio-2-D-methylpropionyl)-L-proline:

In a flask, 30 g of 3-acetylthio-2-D-methylpropionic acid, 0.5 ml of dimethylformamide and 26 g of thionyl chloride were placed, and the mixture was allowed to react at 30°C overnight. After the reaction was over, the reaction mixture was distilled under reduced pressure and a fraction boiling at 79°—85°C at 2—3 mm Hg was collected and cooled to give 29.4 g of 3-acetylthio-2-D-methylpropionyl chloride. A solution of 10 g of the thus-prepared chloride in 100 ml of methylene chloride was added dropwise to 6.4 g of L-proline slurried in 20 ml of N,N-dimethylbenzylamine, while keeping the reaction temperature at 0°C. After the addition was completed, the mixture was allowed to react at 0°C for about 1 hour. After the reaction was over, 100 ml of ice water and 10 ml of concentrated hydrochloric acid were added to the reaction mixture and the methylene chloride layer and the aqueous layer were separated with a separatory funnel. The aqueous layer was extracted twice with 100 ml of methylene chloride. The methylene chloride layers were combined with the one previously obtained and washed with 100 ml of 5% hydrochloric acid and twice with 100 ml of ice water, and dried over anhydrous sodium sulfate overnight.

Thereafter, methylene chloride was distilled away, leaving 129 g of 1-(3-acetylthio-2-D-methylpropionyl)-L-proline as a viscous transparent oily substance. The specific rotation of this oily substance $[\alpha]_D^{29.5}$ was −139° (C = 2.1, 99% ethanol). By treating the oily substance with a mixed solvent of n-hexane and ethyl acetate, colorless crystals with a specific rotation $[\alpha]_D^{25°}$ of −161° (C = 1.5, 99% ethanol) were obtained. 1-(3-Acetylthio-2-D-methylpropionyl)-L-proline was identified by N.M.R.

**Claims**

1. A process for producing optically active carboxylic acid amides of the formula;

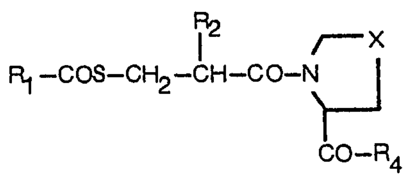

(I)

wherein $R_1$ is an alkyl group, an aralkyl group or an aryl group, $R_2$ is an alkyl group, $R_4$ is a hydroxyl group, an amino group or an alkoxy group, and X is a methylene group or a sulfur atom, which comprises allowing carbothioic acids of the formula;

$$R_1—COSH \quad (II)$$

wherein $R_1$ is as defined above, to react with unsaturated carboxylic acid esters of the formula;

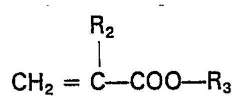

(III)

wherein $R_2$ and $R_3$ each represent an alkyl group, to give esters of the formula;

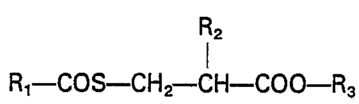

(IV)

wherein $R_1$, $R_2$ and $R_3$ are as defined above, biochemically and asymmetrically hydrolyzing the esters of the formula (IV) above to give optically active carboxylic acids of the formula;

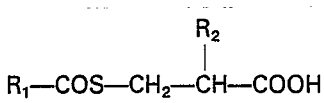

(V)

wherein $R_1$ and $R_2$ are as defined above, and allowing the optically active carboxylic acids of the formula (V) above to react with amino compounds of the formula;

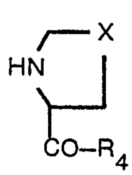

(VI)

wherein $R_4$ and X are as defined above.

2. A process according to Claim 1, wherein the reaction of the carbothioic acids of the formula (II) with the unsaturated carboxylic acid esters of the formula (III) is conducted in the presence of a polymerization inhibitor, the asymmetrical hydrolyzation of the esters of the formula (IV) is conducted by using enzymes or microorganisms under pH 2—11 at 5°—50°C, and the reaction of the optically active carboxylic acids of the formula (V) with the amine compounds of the formula (VI) is conducted in a solvent in the presence of a base at −50°C or higher.

3. A process according to Claim 2 wherein the microorganisms are microorganisms such as genuses Torulopsis, Aspergillus, Bacillus, Phodococcus, Candida, Botrytis, Ophiobolus, Chaetomium, Cladosporium, Pseudomonas, Escherichia, Staphylococcus, Alcaligenes, Streptomyces, Nocardia, Mycobacterium and Agrobacterium.

**Patentansprüche**

1. Ein Verfahren zur Herstellung von optisch aktiven Carbonsäureamiden der Formel

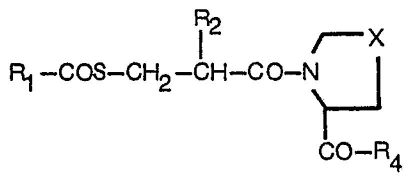

(I)

in der
$R_1$ eine Alkylgruppe, eine Aralkylgruppe oder eine Arylgruppe ist,
$R_2$ eine Alkylgruppe ist,
$R_4$ eine Hydroxylgruppe, eine Aminogruppe oder eine Alkoxygruppe ist und
X eine Methylengruppe oder ein Schwefelatom ist, die Maßnahmen umfassend, Carbothiolsäuren der Formel

$$R_1—COSH \qquad (II)$$

in der $R_1$ wie vorstehend definiert ist, mit ungesättigten Carbonsäureestern der Formel

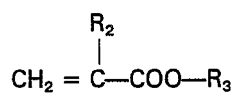

(III)

in der $R_2$ und $R_3$ Alkylgruppen sind, zur Bildung von Estern der Formel

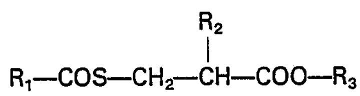

(IV)

in der $R_1$, $R_2$ und $R_3$ wie vorstehend definiert sind, reagieren zu lassen, die Ester der vorstehenden Formel (IV) zur Bildung optisch aktiver Carbonsäuren der Formel

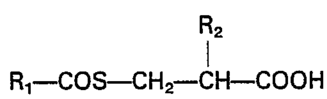

(V)

in der $R_1$ und $R_2$ wie vorstehend definiert sind, biochemisch und asymmetrisch zu hydrolysieren und die optisch aktiven Carbonsäuren der vorstehenden Formel (V) mit Aminoverbindungen der Formel

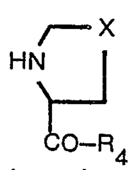

(VI)

in der $R_4$ und X wie vorstehend definiert sind, reagieren zu lassen.

2. Ein Verfahren nach Anspruch 1, bei dem die Umsetzung der Carbothiolsäuren der Formel (II) mit den ungesättigten Carbonsäureestern der Formel (III) in Gegenwart eines Polymerisationsinhibitors, die asymmetrische Hydrolysation der Ester der Formel (IV) under Verwendung von Enzymen oder Mikroorganismen unter pH 2 bis 11 bei 5 bis 50°C und die Umsetzung der optisch aktiven Carbonsäuren der

Formel (V) mit den Aminoverbindungen der Formel (VI) in einem Lösungsmittel in Gegenwart einer Base bei −50°C oder höher durchgeführt werden.

3. Ein Verfahren nach Anspruch 2, bei dem die Mikroorganismen solche der Gattungen Torulopsis, Aspergillus, Bacillus, Phodococcus, Candida, Botrytis, Ophiobolus, Chaetomium, Cladosporium, Pseudomonas, Escherichia, Staphylococcus, Alcaligenes, Streptomyces, Nocardia, Mycobacterium und Agrobacterium sind.

**Revendications**

1. Procédé de préparation d'amides optiquement actifs d'acide carboxylique de formule:

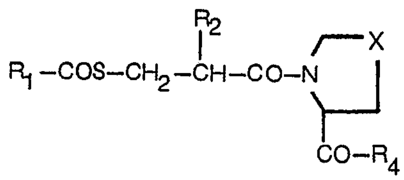

(I)

dans laquelle $R_1$ est un groupe alcoyle, un groupe aralcoyle ou un groupe aryle, $R_2$ est un groupe alcoyle, $R_4$ est un groupe hydroxyle, un groupe amino ou un groupe alcoxy, et X est un groupe méthylène ou un atome de soufre, qui consiste à faire réagir des acides carbothioïques de formula:

$$R_1—COSH \qquad (II)$$

dans laquelle $R_1$ est tel que défini ci-dessus, sur des carboxylates insaturés de formule:

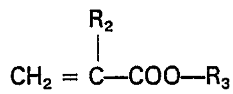

(III)

dans laquelle $R_2$ et $R_3$ représentent un groupe alcoyle, pour obtenir des esters de formule:

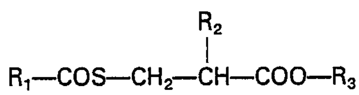

(IV)

dans laquelle $R_1$, $R_2$ et $R_3$ sont tels que définis ci-dessus, à hydrolyser biochimiquement et dissymétriquement les esters de formule (IV) ci-dessus, en des acides carboxyliques optiquement actifs de formule:

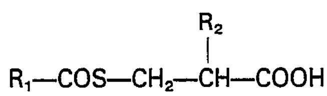

(V)

dans laquelle $R_1$ et $R_2$ sont tels que définis ci-dessus, et à faire réagir les acides carboxyliques optiquement actifs de formule (V) ci-dessus, sur des amines de formule:

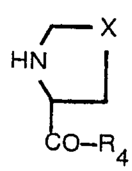

(VI)

dans laquelle $R_4$ et X sont tels que définis ci-dessus.

2. Procédé suivant la revendication 1, qui consiste à effectuer la réaction des acides carbothioïques de formule (II) sur les carboxylates insaturés de formule (III), en la présence d'un inhibiteur de polymérisation, à effectuer l'hydrolysation dissymétrique des esters de formule (IV) en utilisant des enzymes ou des microorganismes à un pH de 2 à 11 et en opérant entre 5 et 50°C, et à effectuer la réaction des acides carboxyliques optiquement actifs de formule (V) sur les amines de formule (VI) dans un solvant, en la présence d'une base, à une température de −50°C ou supérieure à −50°C.

3. Procédé suivant la revendication 2, dans lequel les microorganismes sont des microorganismes tels que des genres Torulopsis, Aspergillus, Bacillus, Phodococcus, Candida, Botrytis, Ophiobolus, Chaetonium, Cladosporium, Pseudomonas, Escherichia, Staphylococcus, Alcaligenes, Streptomyces, Nocardia, Mycobacterium et Agrobacterium.